# EUROPÄISCHE PATENTSCHRIFT

(11) **EP 4 395 592 B1**
(45) Veröffentlichungstag und Bekanntmachung des Hinweises auf die Patenterteilung: **22.07.2026**
(21) Anmeldenummer: 22765553.7
(22) Anmeldetag: 17.08.2022
(51) Int. Cl.: A41C 3/00, A41C 3/10, A41C 3/14, A41C 1/06, A41C 1/02, A61F 13/14

(54) **BUSTIER AUS SILIKON**
SILICONE BUSTIER
BUSTIER EN SILICONE

(30) Priorität: 01.09.2021 DE 102021122657
(43) Veröffentlichungstag der Anmeldung: 10.07.2024
(73) Patentinhaber: Vollmar, Mariam, 95517 Seybothenreuth (DE)
(72) Erfinder: Vollmar, Mariam, 95517 Seybothenreuth (DE)
(74) Vertreter: Hannke Bittner & Partner mbB Regensburg
(86) Internationale Anmeldenummer: PCT/EP2022/073005
(87) Internationale Veröffentlichungsnummer: WO 2023/030895

(56) Entgegenhaltungen:
- WO-A1-2018/186553
- WO-A1-2020/200286
- WO-A2-2012/094264

## Beschreibung

Die vorliegende Erfindung bezieht sich auf einen Kompressions-Büstenhalter bzw. -Bustier.

Die Erfindung wird insbesondere auf einen Kompressions-BH beschrieben, der nach Brustoperationen eingesetzt werden kann, bzw. sollte. Es wird jedoch darauf hingewiesen, dass die Erfindung auch für andere Bekleidungsstücke, beispielsweise Sport-BHs oder auch sonstige Unterbekleidungsstücke für Damen und Herren geeignet ist.

In den vergangenen Jahren haben Brustoperationen stetig zugenommen. Im Rahmen derartiger Brustoperationen werden teilweise Implantate in die Brust eingesetzt, welche mit der Zeit mit dem Hautgewebe verwachsen. Für einen Zeitraum von beispielsweise 6 Wochen nach einer Brustbehandlung wie etwa einer Brustvergrößerung sollten die Nutzerinnen einen Kompressions-BH bzw. -Bustier tragen. Dieses dient dazu, die Brust in der richtigen Form zu halten, und sie während der Heilungsphase zu schützen. Daneben soll es mit einem gleichmäßigen Druck die Wundheilung fördern.

So beschreibt die internationale Patentanmeldung WO 2020/200286 A1 bereits eine angepasste Stützvorrichtung mit einer angepassten tragbaren Struktur analog dem Oberbegriff der vorliegenden Anmeldung. Die Patentanmeldung WO 2020/200286 A1 offenbart den Oberbegriff des Anspruchs 1.

Diese Bekleidungsstücke sind daher für die Nutzerin mit hohem Aufwand und zum Teil auch mit Schmerzen verbunden. Auch sollten diese Kompressions-BHs während der Nacht getragen werden.

Der vorliegenden Erfindung liegt daher die Aufgabe zugrunde, einen entsprechenden Kompressions-BH, im Folgenden auch als "Bustier" bezeichnet zur Verfügung zu stellen, das den Tragekomfort erheblich erleichtert. Dies wird erfindungsgemäß durch den Gegenstand von Anspruch 1 erreicht. Vorteilhafte Ausführungsformen und Weiterbildungen sind Gegenstand der Unteransprüche.

Ein erfindungsgemäßes Bekleidungsstück, insbesondere zur postoperativen Anwendung und insbesondere ein Bustier weist wenigstens eine und bevorzugt zwei Aufnahmeeinrichtungen zur Aufnahme von Körperteilen und insbesondere von Brüsten auf. Weiterhin weist das Bekleidungsstück eine erste dem Körper zugewandte Schicht und wenigstens abschnittsweise eine zweite Schicht und/oder Stützelemente auf, welche wenigstens abschnittsweise an der ersten Schicht (zumindest mittelbar) angeordnet ist.

Erfindungsgemäß weist die erste Schicht eine geringere Härte auf als die zweite Schicht und die erste Schicht ist aus einem Silikonmaterial hergestellt oder weist ein solches auf.

Es wird daher vorgeschlagen, dass eine innere Schicht, welche auf der Haut bzw. der Brust anliegt und/oder welche von der Haut durch eine Abdeckschicht abgegrenzt ist, weicher ist als eine entsprechende zweite Schicht. Dies ist ungewöhnlich, da der Kompressions-BH die Aufgabe haben soll in möglichst zuverlässiger Weise das Gewebe bzw. die Brüste zu stabilisieren.

Die Anmelderin hat jedoch herausgefunden, dass auch eine weichere Innenschicht anwendbar ist, insbesondere wenn diese wiederum durch eine Außenschicht oder durch Verstärkungselemente verstärkt wird.

Damit liegt der Erfindung die Idee zugrunde, das Hautgewebe der Brust im Wesentlichen durch die besagte innere Schicht fortzuführen und erst auf bzw. oberhalb der inneren Schicht eine entsprechende Verstärkung in Form von Verstärkungselementen und/oder der zweiten Schicht anzubringen.

Wie oben erwähnt, ist die vorliegende Erfindung auch auf andere Bekleidungstücke und insbesondere andere Bekleidungsstücke, welche nach Operationen getragen werden können und insbesondere unmittelbar auf der Haut getragen werden können, gerichtet. Die Anmelderin behält sich daher vor, Schutz zu beanspruchen für ein Bekleidungsstück, welches wenigstens einen unmittelbar an der Haut anzuordnenden Abschnitt aufweist, sowie ein Befestigungsmittel, welches diesen Abschnitt an dem Hauptabschnitt hält.

Weiterhin weist das Bekleidungsstück eine erste dem Körper zugewandte Schicht und wenigstens abschnittsweise eine zweite Schicht und/oder Stützelemente auf, welche wenigstens abschnittsweise an der ersten Schicht (zumindest mittelbar) angeordnet ist.

Erfindungsgemäß weist die erste Schicht eine geringere Härte auf als die zweite Schicht und die erste Schicht ist aus einem Silikonmaterial hergestellt oder weist ein solches auf.

Dabei kann dieses Bekleidungsstück zum Stützen von unterschiedlichen Körperteilen dienen, beispielsweise der Beine, der Schenkel, des Bauches oder des Hinterns.

Bevorzugt ist die Dehnbarkeit der ersten Schicht größer als die Dehnbarkeit der zweiten Schicht oder der zweiten Elemente.

Bevorzugt handelt es sich bei den Aufnahmeeinrichtungen um die sog. Cups, welche die weiblichen Brüste aufnehmen. Bevorzugt sind die Aufnahmeeinrichtung derart gestaltet, dass sie wenigstens 50% der Oberfläche der jeweiligen Brust aufnehmen, bevorzugt wenigstens 60%, bevorzugt wenigstens 70% und bevorzugt wenigstens 80% und bevorzugt wenigstens 90%. Bei einer bevorzugten Ausführungsform deckt die erste Schicht die Brust vollständig oder im Wesentlichen vollständig ab.

Weiterhin ist es möglich, dass die Härte der inneren Schicht mit einem Verfahren gemäß Shore-A oder Shore-A00 bestimmbar ist. Dabei ist für die Bestimmung einer Shore-Härte A nach DIN 53505 als Eindringkörper (Identer) ein Kegelstumpf mit einer Stirnfläche von 0,79 +/- 0,01 mm im Durchmesser und einem Öffnungswinkel von 35° +/- 0,25° zu verwenden.

Bevorzugt liegt eine Shore-A-Härte der ersten bzw. innersten Schicht zwischen 0 und 30, bevorzugt zwischen 0 und 20, bevorzugt zwischen 5 und 20, bevorzugt zwischen 5 und 10.

Bevorzugt liegt eine Shore-00-Härte der ersten bzw. inneren Schicht zwischen 45 und 90, bevorzugt zwischen 55 und 85, bevorzugt zwischen 60 und 80.

Bei einer weiteren bevorzugten Ausführungsform weist die zweite bzw. äußere Schicht eine Shore-A-Härte auf, die zwischen 20 und 50, bevorzugt zwischen 25 und 45 und besonders bevorzugt zwischen 30 und 40 liegt. Damit ist hier ein mittlerer Härtegrad der äußeren Schicht vorgesehen.

Es wäre jedoch auch denkbar, dass die äußere Schicht eine große Härte aufweist und insbesondere eine Shore-A-Härte, die größer ist als 40 und bevorzugt größer als 45
Bevorzugt ist an der inneren Schicht und insbesondere an der der Haut zugewandten Oberfläche der inneren Schicht noch eine Abdeckschicht angeordnet. Bevorzugt ist auch diese Abdeckschicht sehr weich, was beispielsweise jedoch auch durch eine geringe Dicke dieser Abdeck-Schicht erreicht werden kann. Damit kommt das Silikon der ersten Schicht bevorzugt nicht unmittelbar auf der Haut zu liegen. Es wäre jedoch auch denkbar, dass die erste Schicht unmittelbar am Körper anliegt.

Bevorzugt weist diese Abdeckschicht eine Dicke auf, die wesentlich geringer ist als die Dicke der ersten Schicht. Bevorzugt beträgt die Dicke der Abdeckschicht weniger als 70% der Dicke der ersten bzw. inneren Schicht, bevorzugt weniger als 60%, bevorzugt weniger als 50%, bevorzugt weniger als 40%, bevorzugt weniger als 30%, bevorzugt weniger als 20%.

Bevorzugt ist die Dehnbarkeit der Abdeckschicht geringer als die Dehnbarkeit der ersten bzw. inneren Schicht.

Bevorzugt ist die zweite Schicht bzw. sind die Stützelemente lediglich abschnittsweise ausgebildet, insbesondere in denjenigen Bereichen, in denen eine Abstützung gewünscht oder gewollt ist. Vorteilhaft sind dabei die zweiten Elemente an die jeweilige Operation bzw. die Operationsergebnisse anpassbar.

Auf diese Weise wird ein Bustier zur Verfügung gestellt, das eine optimale Passform und einen hohen Tragekomfort hat, wobei dies durch ein angenehm weiches Material erreicht wird. Daneben ist die innere Schicht bevorzugt hochelastisch und sehr bequem und erzeugt auch keine Druckstellen und kein Einschnüren. Daneben ist eine sehr gute Hautverträglichkeit gegeben.

Bevorzugt entspricht der Kompressions-BH der Kompressions-Klasse 2 (23 - 30 mm hg). Bevorzugt ist auch die zweite Schicht aus einem Silikon hergestellt. Dabei ist die zweite Schicht wie oben erwähnt härter als die erste Schicht.

Bei einer weiteren vorteilhaften Ausführungsform weist das Bekleidungsstück auf einer dem Körper abgewandten Außenoberfläche eine Schicht und/oder ein Element aus einem weiteren Material auf, insbesondere einem Material, welches sich von den Materialien der ersten und zweiten Schicht unterscheidet. Dabei kann diese Außenoberfläche abschnittsweise direkt an der ersten Schicht und/oder abschnittsweise direkt an der zweiten Schicht anliegen.

Bevorzugt handelt sich bei diesem weiteren Material um ein textiles Gewebe, welches über der ersten oder der zweiten Silikonschicht angeordnet ist. Weiterhin ist es auch möglich, dass die erste und die zweite Schicht im Wesentlichen vollständig an der Außenoberfläche von diesem weiteren Material abgedeckt sind. Bevorzugt ist dieses weitere Material aus einer Gruppe von Materialien ausgewählt, welche Fasermaterialien, Cellulose, Kunststoffe und textile Materialien enthält. Weiterhin kann es sich bei dem Material auch um ein recyclingfähiges Material handeln.

Bei einer weiteren vorteilhaften Ausführungsform sind die Aufnahmeeinrichtungen über wenigstens einen Verbindungsbereich miteinander verbunden und/oder die Aufnahmeeinrichtungen sind lösbar miteinander verbunden. So kann das Bekleidungsstück zwischen den beiden Aufnahmeeinrichtungen, insbesondere den beiden Cups einen Verbindungssteg aufweisen und vorteilhaft auch eine lösbare Verbindung. Vorteilhaft ist dabei diese lösbare Verbindung beispielsweise als Reißverschluss oder als Klettverschluss oder als Schnürverschluss ausgebildet.

Bei einer weiteren bevorzugten Ausführungsform ist das Bustier bügelfrei ausgebildet. Bevorzugt weist das Bustier keine Elemente aus einem biegesteifen Material auf.

Bei einer weiteren vorteilhaften Ausführungsform weist die erste Schicht eine Dicke auf, die größer ist als 2 mm, bevorzugt größer als 3 mm, bevorzugt größer als 4 mm und besonders bevorzugt größer als 5 mm.

Bei einer weiteren vorteilhaften Ausführungsform weist die erste Schicht eine Dicke auf, die geringer ist als 20 mm, bevorzugt geringer als 18 mm, bevorzugt geringer als 15 mm, bevorzugt geringer als 12 mm und besonders bevorzugt geringer als 10 mm.

Bei einer weiteren vorteilhaften Ausführungsform weist die äußere Schicht und/oder weisen die äußeren Elemente eine Dicke auf, die größer sind als 1 mm, bevorzugt größer als 2 mm. Bei einer weiteren bevorzugen Ausführungsform weist die äußere Schicht und/oder weisen die äußeren Elemente eine Dicke auf, die geringer ist als 12 mm, bevorzugt geringer als 10 mm, bevorzugt geringer als 8 mm und bevorzugt geringer als 7 mm. Bevorzugt ist daher die Dicke der inneren Schicht größer als die Dicke der äußeren Schicht.

Erfindungsgemäß ist die erste Schicht aus medizinischem Silikon und/oder Implantat-Silikon hergestellt und/oder die zweite Schicht ist aus einem Kosmetik-Silikon hergestellt. Es wird daher vorgeschlagen, dass für die erste Schicht bevorzugt das gleiche Silikon verwendet wird, welches auch für die Brustimplantate selbst verwendet wird und/oder ein bezüglich diesem insbesondere in Bezug auf die Härte ähnliches Material. So kann beispielsweise für die erste Schicht Silikongel, beispielsweise in einem Festigkeitsgrad 1 verwendet werden.

Durch diese besonders weiche Ausgestaltung des Materials kann eine ideale und schonende Anpassung an die operierte Brust erfolgen. Bevorzugt ist jedoch diese erste Schicht zumindest in Richtung des Körpers wenigstens teilweise und besonders bevorzugt vollständig abgedeckt von der oben erwähnten weiteren Abdeckschicht oder einem weiteren Material.

Dabei ist es möglich, dass diese auf der Haut aufliegende Abdeckschicht des Bekleidungsstückes an die erste Schicht angeklebt ist. Es ist weiterhin möglich, dass die an der Haut anliegende Schicht, d.h. die Abdeckschicht, die insbesondere an der ersten Schicht angeordnet ist, luftdurchlässig ausgebildet ist bzw. eine gewisse Dampfdurchlässigkeit aufweist. Auf diese Weise kann eine gewisse Atmungsaktivität erreicht werden.

Es wäre jedoch auch möglich, dass die an der Haut anliegende Schicht luft- und/oder gas- und/oder dampfundurchlässig ausgebildet ist.

Bei einer weiteren bevorzugten Ausführungsform sind die erste und die zweite Schicht aneinander angeordnet und insbesondere unmittelbar aneinander angeordnet. Dabei ist es möglich, dass die erste und die zweite Schicht miteinander wenigstens abschnittsweise und bevorzugt vollständig verklebt sind. Es kann jedoch auch zwischen der ersten Schicht und der zweiten Schicht eine weitere Schicht, bzw. eine Membran angeordnet sein. Besonders bevorzugt ist die erste Schicht in ihrer Gesamtheit in einer Umhüllung angeordnet und/oder grenzt nicht unmittelbar an die Umgebung. Dabei kann diese Umhüllung aus mehreren Komponenten gebildet sein. Auch können diese mehreren Komponenten miteinander verbunden sein, beispielsweise über eine Naht oder dergleichen. Auch wäre eine Verklebung dieser Komponenten denkbar.

Bei einer weiteren vorteilhaften Ausführungsform ist an der ersten Schicht, insbesondere der dem Körper zugewandten Oberfläche eine Haut-Feel-Beschichtung angeordnet. Hierunter ist insbesondere eine Beschichtung zu verstehen, welche für die Haut angenehm wirkt bzw. der Haut das Gefühl gibt, ein weiteres Hautelement ist hieran angeordnet.

Vorteilhaft weist diese Beschichtung eine Dicke auf, die größer ist als 0,05 mm, bevorzugt größer als 0,1 mm, bevorzugt größer als 0,2 mm.

Bei einer weiteren vorteilhaften Ausführungsform weist diese Beschichtung (bei der es sich auch um die oben erwähnte Abdeckschicht handeln kann) eine Dicke auf, die geringer ist als 1,0 mm, bevorzugt geringer als 0,8 mm und besonders bevorzugt geringer als 0,6 mm.

Bei einer weiteren vorteilhaften Ausführungsform weist das Bekleidungsstück eine Stützeinrichtung zum Stützen der Brust oder der Brüste auf. Vorteilhaft ist diese Stützeinrichtung als Randabschnitt ausgebildet. Dabei kann bevorzugt diese Stützeinrichtung im Wesentlichen geradlinig verlaufen. Vorteilhaft weist die Stützeinrichtung gurtartige Kanten und insbesondere auch runde Kanten auf, welche auch Handförmig unterhalb der Brust zu liegen kommen.

Bevorzugt erstreckt sich diese Stützeinrichtung in einem angelegten Zustand des Bustiers im Wesentlichen senkrecht zur Körperachse der das Bustier tragenden Person.

Bei einer weiteren vorteilhaften Ausführungsform weist das Bekleidungsstück Schulterträger auf. Dabei können diese Schulterträger öffenbar sein und beispielsweise über Klettverschlüsse verschlossen sein.

Bei einer bevorzugten Ausführungsform weist das Bekleidungsstück weitere Verschlusseinrichtungen wie Klettverschlüsse auf, die eine weitere und engere Einstellung des Bekleidungsstücks ermöglichen.

Bei einer weiteren vorteilhaften Ausführungsform weisen die äußere Schicht und/oder die äußeren Elemente wenigstens abschnittsweise eine keilförmige und/oder polygonale und insbesondere dreieckige Form auf. Bevorzugt weist die äußere Schicht und/oder weisen die äußeren Elemente eine sich ändernde Dicke auf.

Auf diese Weise kann ein sanfter Druck auf das Gewebe und an das Gewebe von der Seite nach oben und innen ausgeübt werden. Auf diese Weise kann ein seitliches Auswuchten der Brust im Heilungsprozess verhindert werden und zusätzlich kann auch in sanfter Weise Lympfstaus vorgebeugt werden.

Bei einer vorteilhaften Ausführungsform verlaufen Randbereiche und/oder Kanten, welche sich in einem an die Benutzerin angelegten Zustand des Bustier im Unterarm-Bereich befinden in einem Winkel, der zwischen 30° und 60°, bevorzugt zwischen 35° und 55° und bevorzugt zwischen 40° und 50° und besonders bevorzugt zwischen unter ungefähr 45°. Diese Gradangaben sind dabei gegenüber einer horizontalen Richtung bzw. senkrecht zu einer Körperachse der das Bustier tragenden Person definiert. Auf diese Weise ergibt sich ein höriger Verlauf und es wird ein Aufrollen verhindert und auch der Tragekomfort erhöht.

Bei einer weiteren vorteilhaften Ausführungsform ist in vorgegebenen ersten Bereichen des Bekleidungsstücks nur eine Schicht und insbesondere nur die erste bzw. dem Körper zugewandte Schicht ausgebildet und in anderen Bereichen beide Schichten. Bevorzugt ist in den genannten ersten Bereichen nur die erste Schicht ausgebildet und in den anderen Bereichen beide Schichten.

So kann insbesondere im Rückenbereich des Bekleidungsstücks nur die erste Schicht ausgebildet sein, nicht aber die zweite Schicht. Auch in den Bereichen der Gurte ist es möglich, dass nur die erste Schicht oder nur die zweite Schicht ausgebildet ist.

Bevorzugt ist in all denjenigen Bereichen, in denen keine Operation stattgefunden hat, bzw. keine Operationsnarben vorhanden sind und die entsprechend nicht gestützt werden müssen nur eine Schicht vorhanden. Bevorzugt ist jedoch in demjenigen Bereich, in dem nur eine Schicht vorhanden ist, diese innerhalb eines Textils angeordnet. Bevorzugt ist in diesen Bereichen zumindest auch zwischen der ersten Schicht und dem Körper die oben beschriebene Abdeckschicht vorhanden.

Weitere Ausführungsformen und Weiterbildungen ergeben sich aus den beigefügten Zeichnungen:
Darin zeigen:
- Fig. 1: eine Darstellung eines Kompressions-BHs nach dem Stand der Technik;
- Fig. 2: eine Darstellung eines erfindungsgemäßen Kompressions-BHs in einer Vorderansicht;
- Fig. 3: eine Darstellung des Kompressions-BHs in einer Rückansicht;
- Fig. 4: ein Aufbau eines erfindungsgemäßen Kompressions-BHs.

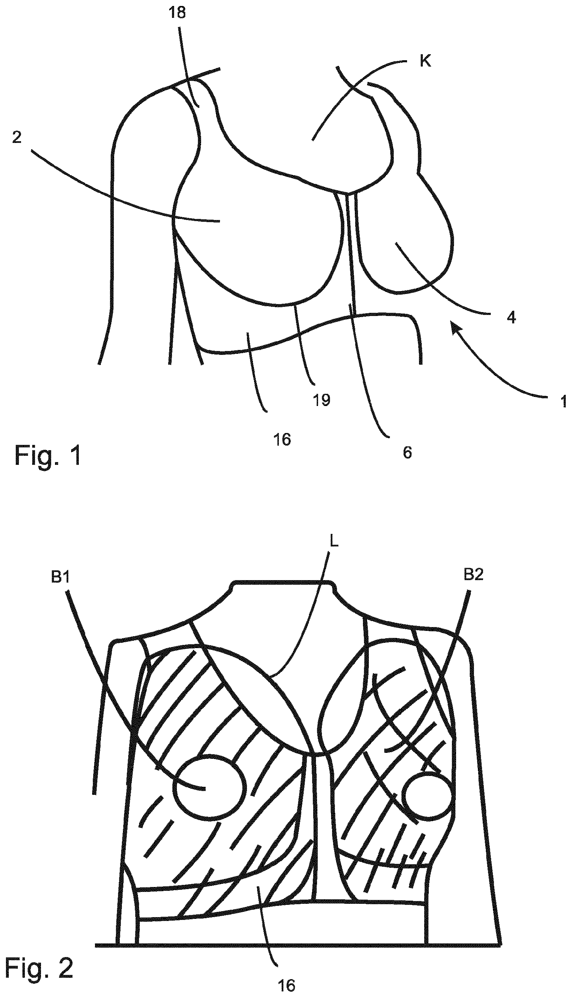

Figur 1 zeigt eine Darstellung eines erfindungsgemäßen Kompressions-BHs bzw. Bustiers an einem Körper. Dabei kennzeichnen die Bezugszeichen 2 und 4 die beiden Aufnahmeeinrichtungen bzw. Cups für die beiden Brüste. Zwischen diesen Aufnahmeeinrichtungen 2,4 ist ein Verschließmittel 6 wie etwa ein Reißverschluss oder ein Klettverschluss angeordnet. Unterhalb der Aufnahmeeinrichtungen befindet sich ein Verstärkungsrand 16, der dazu dient, die Aufnahmeeinrichtungen und damit die Brüste zu stützen. Das Bezugszeichen 18 kennzeichnet einen Schulterträger, der ebenfalls bevorzugt als Klettverschluss ausgebildet ist und daher geöffnet werden kann.

Das Bezugszeichen 19 kennzeichnet einen Stützrand, der gekrümmt ausgebildet ist und an der Unterseite der zu stützenden Brust anliegt. Dieser ist derart geformt, dass er die jeweilige Brust wie eine Hand stützt.

Figur 2 zeigt ein erfindungsgemäßes Bustier an einem Körper. Dabei kennzeichnet das Bezugszeichen B1 einen ersten Bereich und das Bezugszeichen B2 einen zweiten Bereich. In dem ersten Bereich B1 ist nur eine Schicht vorgesehen, in dem zweiten Bereich B2 zwei Schichten. Dargestellt ist auch wieder der Stützrand 16, der wie hier erkennbar ist, gekrümmt ausgeführt ist, um zusätzlichen Halt des Bustiers zu geben. Das Bezugszeichen L kennzeichnet eine Linie, welche darstellt, wie die Aufnahmeeinrichtungen im Falle des erfindungsgemäßen Bustiers im Vergleich zu herkömmlichem Bustier verändert ist. Auf diese Weise decken die Aufnahmeeinrichtungen die beiden Brüste im Wesentlichen vollständig ab.

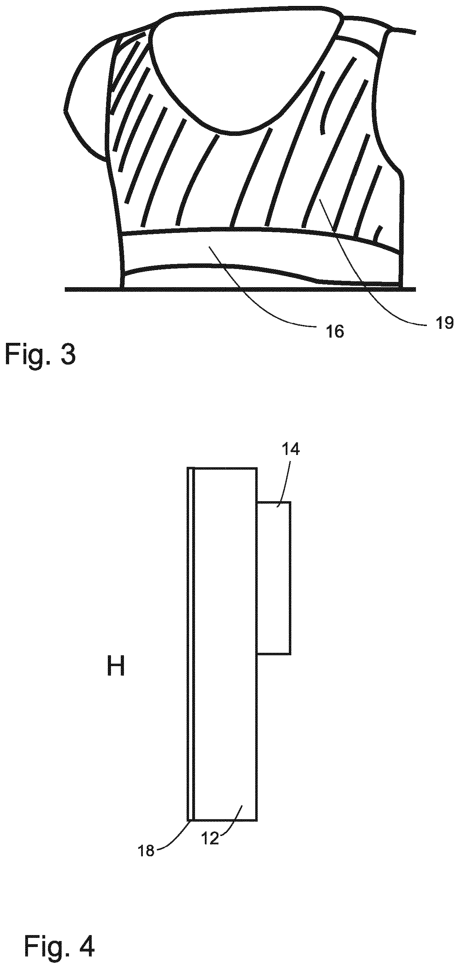

Figur 3 zeigt eine Rückenansicht des in Figur 2 gezeigten Bustiers. Auch hier ist wieder der umlaufende Rand 16 dargestellt. Im Rückenbereich weist bevorzugt das Bustier bzw. dessen Rückenpartier bevorzugt nur eine Schicht und insbesondere nur die erste Schicht auf.

Figur 4 zeigt einen Aufbau eines erfindungsgemäßen Bustiers in schematischer Darstellung. Dabei bezieht sich das Bezugszeichen 12 auf die erste Schicht, die wie oben erwähnt bevorzugt aus einem Implantat-Silikon hergestellt ist. Das Bezugszeichen 14 kennzeichnet die zweite Schicht, die hier unmittelbar an der ersten Schicht angeordnet ist, beispielsweise über eine (nicht gezeigte) Verklebung. Das Bezugszeichen 18 kennzeichnet eine Abdeckschicht, die an der der Haut H zugewandten Seite auf der ersten Schicht 12 angeordnet ist. Diese Abdeckschicht weist dabei eine wesentlich geringere Dicke auf als die erste Schicht 12. Bevorzugt ist die erste Schicht wenigstens 2x so dick, bevorzugt wenigstens 4x so dick, bevorzugt wenigstens 5x so dick und bevorzugt wenigstens 6x so dick als die Abdeckschicht 18. Die Abdeckschicht ist bevorzugt flexibel.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind. Es wird weiterhin darauf hingewiesen, dass in den einzelnen Figuren auch Merkmale beschrieben wurden, welche für sich genommen vorteilhaft sein können. Der Fachmann erkennt unmittelbar, dass ein bestimmtes in einer Figur beschriebenes Merkmal auch ohne die Übernahme weiterer Merkmale aus dieser Figur vorteilhaft sein kann. Ferner erkennt der Fachmann, dass sich auch Vorteile durch eine Kombination mehrerer in einzelnen oder in unterschiedlichen Figuren gezeigter Merkmale ergeben können.

## Patentansprüche

1. Bekleidungsstück (1) insbesondere zur post-operativen Anwendung, insbesondere Bustier mit Aufnahmeeinrichtungen (2, 4) zur Aufnahme von Körperteilen und insbesondere von Brüsten, mit einer ersten, dem Körper zugewandten Schicht (12) und wenigstens abschnittsweise einer zweiten Schicht (14) und Stützelementen (14), welche wenigstens abschnittsweise an der ersten Schicht (12) angeordnet ist, wobei die erste Schicht aus einem Silikonmaterial hergestellt ist,
**dadurch gekennzeichnet, dass**
die erste Schicht (12) eine geringere Härte aufweist als die zweite Schicht (14), wobei die erste Schicht aus medizinischem Silikon und/oder Implantatsilikon hergestellt ist und/oder die zweite Schicht aus einem Kosmetiksilikon hergestellt ist.

2. Bekleidungsstück nach Anspruch 1,
**dadurch gekennzeichnet, dass**
die zweite Schicht (14) aus einem Silikon hergestellt ist.

3. Bekleidungsstück nach wenigstens einem der vorangegangenen Ansprüche,
**dadurch gekennzeichnet, dass**
das Bekleidungsstück an einer dem Körper abgewandten Außenoberfläche ein weiteres Material aufweist.

4. Bekleidungsstück (1) nach dem vorangegangenen Anspruch,
**dadurch gekennzeichnet, dass**
das weitere Material aus einer Gruppe von Materialien ausgewählt ist, welche Fasermaterialien, Zellulose, Kunststoffe und textile Materialien enthält.

5. Bekleidungsstück nach wenigstens einem der vorangegangenen Ansprüche,
**dadurch gekennzeichnet, dass**
die Aufnahmeeinrichtungen über einen Verbindungsbereich miteinander verbunden sind und/oder dass die Aufnahmeeinrichtungen lösbar miteinander verbunden sind.

6. Bekleidungsstück nach wenigstens einem der vorangegangenen Ansprüche,
**dadurch gekennzeichnet, dass**
die erste Schicht (12) eine Dicke aufweist, die größer ist als 2mm, bevorzugt größer als 3mm, bevorzugt größer als 4mm und bevorzugt größer als 5mm und/oder dass die erste Schicht eine Dicke aufweist, die geringer ist als 20mm, bevorzugt geringer als 18mm, bevorzugt geringer als 15mm, bevorzugt geringer als 12mm und bevorzugt geringer als 10mm.

7. Bekleidungsstück nach wenigstens einem der vorangegangenen Ansprüche,
**dadurch gekennzeichnet, dass**
die äußere Sicht und/oder die äußeren Elemente eine Dicke aufweisen, die größer ist als 1mm, bevorzugt größer als 2mm und/oder dass die äußere Schicht und/oder die äußeren Elemente eine Dicke aufweisen, die kleiner ist als 12mm, bevorzugt geringer als 10mm und bevorzugt geringer als 7mm.

8. Bekleidungsstück nach wenigstens einem der vorangegangenen Ansprüche,
**dadurch gekennzeichnet, dass**
die erste Schicht (12) und die zweite Schicht (14) aneinander angeordnet sind.

9. Bekleidungsstück nach wenigstens einem der vorangegangenen Ansprüche,
**dadurch gekennzeichnet, dass**
das Bekleidungsstück eine Stützeinrichtung zum Stützen der Brust aufweist.

10. Bekleidungsstück nach wenigstens einem der vorangegangenen Ansprüche,
**dadurch gekennzeichnet, dass**
die äußere Schicht und/oder die äußeren Elemente wenigstens abschnittweise eine keilförmige und/oder eine polygonale und insbesondere dreieckige Form aufweisen.

11. Bekleidungsstück nach wenigstens einem der vorangegangenen Ansprüche,
**dadurch gekennzeichnet, dass**
Randbereiche und/oder Kanten, welche sich in einem an die Benutzerin angelegten Zustand im Unterarmbereich befinden, in einem Winkel verlaufen, der zwischen 30° und 60°, bevorzugt zwischen 35° und 55°, bevorzugt zwischen 40° und 50° und besonders bevorzugt bei etwa 45° verlaufen.

12. Bekleidungsstück nach wenigstens einem der vorangegangenen Ansprüche,
**dadurch gekennzeichnet, dass**
an der ersten, dem Körper zugewandten Schicht eine Abdeckschicht angeordnet ist.

13. Bekleidungsstück nach wenigstens einem der vorangegangenen Ansprüche,
**dadurch gekennzeichnet, dass**
in vorgegebenen Bereichen des Bekleidungsstücks (B1) nur eine Schicht (12, 14) ausgebildet ist und in anderen Bereichen beide Schichten (12, 14).

## Claims

1. Garment (1), in particular for post-operative use, specifically a bustier having receiving formations (2, 4) for accommodating body parts and in particular breasts, comprising a first layer (12) facing the body and, at least in sections, a second layer (14) and support elements (14) arranged at least in sections on the first layer (12), wherein the first layer is made of a silicone material,
**characterized in that**
the first layer (12) has a lower hardness than the second layer (14), wherein the first layer is made of medical-grade silicone and/or implant-grade silicone and/or the second layer is made of cosmetic-grade silicone.

2. Garment according to claim 1, **characterized in that** the second layer (14) is made of a silicone.

3. Garment according to at least one of the preceding claims,
**characterized in that**
the garment comprises a further material on an outer surface facing away from the body.

4. Garment (1) according to the preceding claim,
**characterized in that**
the further material is selected from a group of materials comprising fiber materials, cellulose, plastics, and textile materials.

5. Garment according to at least one of the preceding claims,
**characterized in that**
the receiving means are connected to one another via a connection region and/or the receiving means are detachably connected to one another.

6. Garment according to at least one of the preceding claims,
**characterized in that**
the first layer (12) has a thickness greater than 2 mm, preferably greater than 3 mm, preferably greater than 4 mm, and preferably greater than 5 mm, and/or that the first layer has a thickness less than 20 mm, preferably less than 18 mm, preferably less than 15 mm, preferably less than 12 mm, and preferably less than 10 mm.

7. Garment according to at least one of the preceding claims,
**characterized in that**
the outer layer and/or the outer elements have a thickness greater than 1 mm, preferably greater than 2 mm, and/or that the outer layer and/or the outer elements have a thickness less than 12 mm, preferably less than 10 mm, and preferably less than 7 mm.

8. Garment according to at least one of the preceding claims, **characterized in that** the first layer (12) and the second layer (14) are arranged against one another.

9. Garment according to at least one of the preceding claims,
**characterized in that**
the garment comprises a support device for supporting the breast.

10. Garment according to at least one of the preceding claims,
**characterized in that**
the outer layer and/or the outer elements have, at least in sections, a wedge-shaped and/or a polygonal, and in particular triangular, shape.

11. Garment according to at least one of the preceding claims,
**characterized in that**
marginal regions and/or edges, which are located in the forearm region when the garment is worn by the user, extend at an angle of between 30° and 60°, preferably between 35° and 55°, preferably between 40° and 50°, and particularly preferably at approximately 45°.

12. Garment according to at least one of the preceding claims,
**characterized in that**
a covering layer is arranged on the first layer facing the body.

13. Garment according to at least one of the preceding claims,
**characterized in that**
only one layer (12, 14) is formed in predetermined regions of the garment (B1), and both layers (12, 14) are formed in other regions.

## Revendications

1. Vêtement (1), en particulier pour un usage post-opératoire, notamment un bustier comportant des zones de réception (2, 4) destinées à accueillir des parties du corps et en particulier les seins, comprenant une première couche (12) orientée vers le corps et, au moins par endroits, une seconde couche (14) ainsi que des éléments de soutien (14) disposés au moins par endroits sur la première couche (12), la première couche étant constituée d'un matériau à base de silicone,
**caractérisé en ce que**
la première couche (12) présente une dureté inférieure à celle de la seconde couche (14), la première couche étant constituée de silicone de qualité médicale et/ou de silicone de qualité « implant » et/ou la seconde couche étant constituée de silicone de qualité cosmétique.

2. Vêtement selon la revendication 1,
**caractérisé en ce que**
la seconde couche (14) est constituée de silicone.

3. Vêtement selon l'une au moins des revendications précédentes,
**caractérisé en ce que**
le vêtement comprend un matériau supplémentaire sur une surface extérieure orientée à l'opposé du corps.

4. Vêtement (1) selon la revendication précédente,
**caractérisé en ce que**
le matériau supplémentaire est choisi dans un groupe de matériaux comprenant des matériaux fibreux, de la cellulose, des matières plastiques et des matériaux textiles.

5. Vêtement selon l'une au moins des revendications précédentes,
**caractérisé en ce que**
les moyens de réception sont reliés entre eux par une zone de liaison et/ou **en ce que** les moyens de réception sont reliés entre eux de manière amovible.

6. Vêtement selon l'une au moins des revendications précédentes,
**caractérisé en ce que**
la première couche (12) présente une épaisseur supérieure à 2 mm, de préférence supérieure à 3 mm, de préférence supérieure à 4 mm et de préférence supérieure à 5 mm, et/ou **en ce que** la première couche présente une épaisseur inférieure à 20 mm, de préférence inférieure à 18 mm, de préférence inférieure à 15 mm, de préférence inférieure à 12 mm et de préférence inférieure à 10 mm.

7. Vêtement selon l'une au moins des revendications précédentes,
**caractérisé en ce que**
la couche extérieure et/ou les éléments extérieurs présentent une épaisseur supérieure à 1 mm, de préférence supérieure à 2 mm, et/ou **en ce que** la couche extérieure et/ou les éléments extérieurs présentent une épaisseur inférieure à 12 mm, de préférence inférieure à 10 mm et de préférence inférieure à 7 mm.

8. Vêtement selon l'une au moins des revendications précédentes,
**caractérisé en ce que**
la première couche (12) et la seconde couche (14) sont disposées l'une contre l'autre.

9. Vêtement selon l'une au moins des revendications précédentes,
**caractérisé en ce que**
le vêtement comprend un dispositif de soutien pour soutenir le sein.

10. Vêtement selon l'une au moins des revendications précédentes,
**caractérisé en ce que**
la couche extérieure et/ou les éléments extérieurs présentent, au moins par endroits, une forme en coin et/ou une forme polygonale, et en particulier triangulaire.

11. Vêtement selon l'une au moins des revendications précédentes,
**caractérisé en ce que**
des zones de bord et/ou des bords, situés dans la zone des aisselles lorsque le vêtement est porté par l'utilisateur, s'étendent selon un angle compris entre 30° et 60°, de préférence entre 35° et 55°, de préférence entre 40° et 50°, et de manière particulièrement préférée à environ 45°.

12. Vêtement selon l'une au moins des revendications précédentes,
**caractérisé en ce que**
une couche de recouvrement est disposée sur la première couche, laquelle est tournée vers le corps.

13. Vêtement selon l'une au moins des revendications précédentes,
**caractérisé en ce que**
une seule couche (12, 14) est formée dans des zones prédéterminées du vêtement (B1), et les deux couches (12, 14) sont formées dans d'autres zones.
